# EUROPÄISCHE PATENTSCHRIFT

(11) **EP 2 114 363 B1**
(45) Veröffentlichungstag und Bekanntmachung des Hinweises auf die Patenterteilung: **18.12.2013**
(21) Anmeldenummer: 08708229.3
(22) Anmeldetag: 25.01.2008
(51) Int. Cl.: A61K 8/64, A61K 8/73, A61Q 19/08, A61K 8/19

(54) **ANTIFALTEN-KOSMETIKUM AUF BASIS VON PEPTIDEN**
ANTI-WRINKLE COSMETIC BASED ON PEPTIDES
PRODUIT COSMÉTIQUE ANTIRIDES À BASE DE PEPTIDES

(30) Priorität: 26.01.2007 DE 102007004916
(43) Veröffentlichungstag der Anmeldung: 11.11.2009
(73) Patentinhaber: Coty Germany GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Gulde Hengelhaupt Ziebig & Schneider
(86) Internationale Anmeldenummer: PCT/EP2008/050905
(87) Internationale Veröffentlichungsnummer: WO 2008/090226

(56) Entgegenhaltungen:
- DE-A1-102005 063 179

## Beschreibung

Die Erfindung betrifft ein Antifalten-Kosmetikum auf Basis von Peptiden, das eine besonders schnelle Wirksamkeit zeigt.

Aus der WO 98/25584 ist ein kosmetisches Präparat mit entzündungswidrigen Eigenschaften bekannt, bestehend aus einer Kombination eines Peptides [Lip]X-His-Phe-Arg-Y mit einem SODhaltigem Enzym- und Vitamingemisch.

Die EP 1 429 716 B1 beansprucht eine kosmetische Zubereitung mit Antifaltenwirkung, die ein Pentapeptid Lys-Thr-Thr-Lys-Ser enthält, das z.B. aus Chlorella-Algen gewonnen wird.

Aus der WO 2006/101855 ist eine Zusammensetzung bekannt, die gegen lichtgeschädigte und alternde Haut eingesetzt wird, die einen Peptid-Mangan-Komplex aus Mangan und 3 Aminosäuren enthält, z.B. Gly-His-Lys-Mn oder Val-His-Lys-Mn sowie zusätzlich Retinol und gegebenenfalls weitere Wirkstoffe. Die Wirkung wurde in vitro durch Stimulation der Kollagensynthese in Fibroblasten bzw. Fibroblastenzunahme ermittelt.

Der Erfindung liegt die Aufgabe zugrunde, ein Antifalten-Kosmetikum bereitzustellen, das seine Wirksamkeit besonders schnell, d.h. in wenigen Tagen Behandlungsdauer bereits deutlich zeigt.

Das erfindungsgemäße Antifalten-Kosmetikum ist dadurch gekennzeichnet, dass es ein Peptidgemisch aus zwei unterschiedlichen Hexapeptiden mit den Aminosäuresequenzen Val-Gly-Val-Ala-Pro-Gly und Phe-Val-Ala-Pro-Phe-Pro und einem Dipeptid mit der Sequenz Tyr-Arg umfasst sowie weiterhin einen Gehalt an Hyaluronsäure, ein oder mehrere Antioxidationsmittel und ein oder mehrere Pigmente mit einer Teilchengröße im Bereich von 0,5-300 µm. Darüber hinaus umfasst das Kosmetikum kosmetische Trägerstoffe, Hilfsstoffe und Gemische davon.

Ein bevorzugtes Hexapeptid Val-Gly-Val-Ala-Pro-Gly ist ein Palmitoyl-VGVAPG-Peptid.

Ein bevorzugtes Dipeptid ist ein N-Acetyl-Tyr-Arg-Hexadodecylester.

Von den eingesetzten Peptiden ist für jedes einzelne eine bestimmte Anti-Alterungswirkung zu erwarten, die allgemein im Bereich von 30-60 Tagen liegt und dabei bis zu 36% Faltenverringerung liegen kann.

Von Hyaluronsäure ist bekannt, dass sie vor allem wasserbildende Eigenschaften hat und als Feuchthaltemittel in kosmetischen Zubereitungen eingesetzt wird.

Antioxidationsmittel sind in vielen Kosmetika enthalten, da sie in der Lage sind, freie Radikale zu binden und damit einhergehenden nachteiligen Wirkungen, z.B. durch UV-Strahlen, auf die Haut zu verringern.

Vorteilhafte Antioxidationsmittel sind aus der Gruppe ausgewählt, bestehend aus Ascorbinsäure, Ascorbylacetat, Ascorbylphosphat, Ascorbylpalmitat, Magnesiumascorbylphosphat, Retinol, Retinolpalmitat, Folsäure, N5-Formyltetrahydrofolsäure, N10-Formyltetrahydrofolsäure, Folsäurekonjugate mit 2-8 Glutamylresten, Tocopherol, Tocopherylacetat, Tocopheryllactat, Tocopherylpalmitat, Tocopherylsuccinat, Tocopherylnicotinat, Tocopheryllinoleat, Flavone, Flavonoide, Histidin, Glycin, Tyrosin, Tryptophan, cis-Urocaninsäure, trans-Urocaninsäure, D,L-Carnosin, D-Carnosin, L-Carnosin, α-Carotin, β-Carotin, Lycopin, Harnsäure, Citronensäure, Milchsäure, Apfelsäure, Palmitinsäure, Phytinsäure, Lactoferrin, Ferulasäure, Glutathion, Cystein, Cystin, Pflanzenextrakte, Gemische von Pflanzenextrakten und Gemische von mehreren dieser Antioxidationsmittel.

Bevorzugte Antioxidationsmittel sind Ascorbinsäure, Ascorbylacetat, Ascorbylphosphat, Ascorbylpalmitat, Magnesiumascorbylphosphat, Retinol, Retinolpalmitat, Tocopherol, Tocopherylacetat, Tocopheryllactat, Tocopherylpalmitat, Tocopherylsuccinat, Tocopherylnicotinat, Tocopheryllinoleat, Flavone, Flavonoide, Pflanzenextrakte, Gemische von Pflanzenextrakten und Gemische von zwei oder mehreren dieser Antioxidationsmittel. Andere Vitamine sind nicht geeignet.

Bekannte Flavonoide sind beispielsweise Naringin, α-Glucosylrutin, α(-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid.

Ein besonders bevorzugtes Antioxidationsmittel ist ein Gemisch von Pflanzenextrakten und Vitaminen, speziell ein Gemisch von Pflanzenextrakten.

Ein ganz besonders bevorzugtes Antioxidationsmittel ist ein Gemisch mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie mit einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser. Ein solches Antioxidationsmittel ist in WO 999/66881 beschrieben.

Weiterhin besonders bevorzugte Antioxidationsmittel sind Calendula-Extrakt, Grüntee-Extrakt, Kaffeebohnen-Extrakt oder deren Kombination.

Der Anteil des Antioxidationsmittels liegt allgemein im Bereich von 6, 1 bis 5 Gew-%. Vorzugsweise ist das Antioxidationsmitted mit 0,3-2 Gew-% enthalten, bezogen auf das Gesamtgewicht des Kosmetikums.

Das erfindungsgemäße Kosmetikum umfasst weiterhin ein Pigment oder Pigmentgemisch, wobei das Pigment aus der Gruppe ausgewählt ist, bestehend aus Titanoxid, Zinkoxid, Aluminiumoxid, Siliziumoxid, Eisenoxide, Manganoxid, Glimmer, Kaolin, manganhaltige Tone, Calciumcarbonat, Talkum, Glimmer-Titanoxid, Glimmer-Titanoxid-Eisenoxid beschichtete Pigmente. Besonders bevorzugt sind Titanoxid, Glimmer, und Kaolin. Weiterhin bevorzugt sind Pigmentgemische mit unterschiedlichen Teilchengrößen, wie z.B. solche, die sich um mindestens 50 µm bis 250 µm unterscheiden, vorzugsweise um 50 µm bis 250 µm, besonders bevorzugt um mindestens 100 µm bis 250 µm, ganz besonders bevorzugt um 100 µm bis 250 µm. In einer anderen Ausführungsform unterscheiden sich die Teilchengrößen um mindestens den Faktor 3, vorzugsweise um mindestens den Faktor 5, besonders bevorzugt um mindestens den Faktor 10. Es versteht sich, dass die Pigmentgemische mindestens zwei Pigmente enthalten, die wiederum identisch oder verschieden hinsichtlich nicht die Teilchengröße betreffende Eigenschaften sein können. Das Pigmentgemisch besteht vorzugsweise aus zwei Pigmenten, ganz besonders bevorzugt aus zwei verschiedenen Pigmenten. Sofern das Pigmentgemisch mehr als zwei Pigmente aufweist, ist es im Sinne der Erfindung ausreichend, wenn mindestens zwei Pigmente eine unterschiedliche Teilchengröße haben, die insbesondere im vorgenannten Bereich differiert. Es ist bevorzugt, wenn sämtliche Pigmente unterschiedliche Teilchengrößen besitzen, wobei in einer ganz besonderen Ausführungsform sich sämtliche Pigmente untereinander um mindestens 50 µm bis 250 µm unterscheiden. Die unterschiedlichen Teilchengrößen gleicher oder verschiedener Pigmente in dem erfindungsgemäßen Kosmetikum, zusammen mit den Wirkstoffen, tragen besonders zu der schnellen Wirksamkeit des Kosmetikums bei.

Der Anteil des Pigments oder Pigmentgemisches liegt im Bereich von 0,05 bis 2 Gew-%, vorzugsweise 0,5-1,2 Gew-%, bezogen auf das Gesamtgewicht des Kosmetikums. Ein bevorzugter Bereich für die Teilchengröße (mittlere Teilchengröße D₅₀) des Pigments liegt bei 1 bis 60 µm. Diese Teilchengröße im µm-Bereich hat sich als besonders vorteilhaft für das Ausfüllen von Hohlräumen, die Anlagerung in Falten und deren Abdeckung erwiesen, insbesondere bei Lichteinfall.

Der Anteil des Peptidgemisches im erfindungsgemäßen Kosmetikum liegt im Bereich von 0,5 bis 18 Gew-%, vorzugsweise 0,9-3 Gew-%, bezogen auf das Gesamtgewicht des Kosmetikums. Dabei kann der Anteil des Peptids Val-Gly-Val-Ala-Pro-Gly im Bereich von 0,01 bis 5 Gew-% liegen, vorzugsweise 0,1 bis 3 Gew-%, bezogen auf das Gesamtgewicht des Kosmetikums.

Der Anteil des Peptids Phe-Val-Ala-Pro-Phe-Pro kann im Bereich von 0,01 bis 8 Gew-% liegen, vorzugsweise 0,1 bis 5 Gew- , besonders bevorzugt 0,1 bis 3 Gew-%, bezogen auf das Gesamtgewicht des Kosmetikums.

Der Anteil des Peptids Tyr-Arg kann im Bereich von 0,01 bis 5 Gew- liegen, vorzugsweise 0,1 bis 3 Gew-%, bezogen auf das Gesamtgewicht des Kosmetikums.

Der Anteil der Hyaluronsäure liegt im Bereich von 0,01 bis 10 Gew- , vorzugsweise 0,5 bis 3 Gew-%, bezogen auf das Gesamt-gewicht des Kosmetikums. Besonders bevorzugt ist der Bereich von 0,5 bis 2 Gew-%.

Für das erfindungsgemäße Kosmetikum ist aufgrund der Einzelsubstanzen eine gewisse Antifaltenwirkung zu erwarten gewesen, die nach mehrfacher Anwendung im Zeitraum von 1 bis 2 Monaten auftreten sollte. Überraschenderweise wurde gefunden, dass die erfindungsgemäße Kombination von Wirk- und Füllstoffen bereits innerhalb weniger Tage, d.h. in etwa 7 bis 10 Tagen Faltenreduzierungen insbesondere der kleinen Falten um Auge, Nase und Mund der Testperson bis zu etwa 50% gegenüber dem Anfangszustand hervorruft. Diese außerordentlich schnelle Wirksamkeit war keineswegs aus den Informationen über die Einzelpeptide bzw. die anderen Bestandteile herleitbar.

Ebenso wenig war diese hervorragende Wirkung aus der in DE 10 2005 063 179 A1 implizit enthaltenen möglichen Kombination der vorgenannten Bestandteile ersichtlich. Erst die Auswahl eines die Kollagensynthese stimulierenden Wirkstoffs (VGVAPG), die Auswahl von zwei kosmetischen Wirkstoffen (Hexapeptide-11 und Dipeptid TR), die Auswahl eines feuchtigkeitsspendenden Wirkstoffs (Hyaluronsäure) und die Auswahl eines Zusatzstoffs (Antioxidantionsmittel) aus vier umfangreichen Listen und in Verbindung mit Pigmenten, die keine Nanopigmente sind, führte zu der wertvollen Beschleunigung der Faltenreduktion, die der allgemeinen Offenbarung der Antifaltenbehandlung ohne zeitlichen Rahmen aus DE 10 2005 063 179 A1 überlegen ist.

Wie die Erfinder des Weiteren unerwartet zeigen konnten, ist die synergistische Interaktion von Vitamin B6 bzw. Vitamin-B6-Komponenten mit weiteren Wirkstoffen, auf die DE 10 2005 063 179 A1 in der kosmetischen Zusammensetzung abstellt, entbehrlich. Insbesondere sind Vitamin B6 bzw. Vitamin-B6-Komponenten als Hilfsstoffe im Sinne der Erfindung nicht notwendig, um die erfindungsgemäße Steigerung der Geschwindigkeit bei der Hautglättung zu erreichen.

Das erfindungsgemäße Antifalten-Kosmetikum kann weitere Hilfsstoffe enthalten, z.B. solche, ausgewählt aus einer Gruppe, bestehend aus anorganische Lichtschutzmittel, organische Lichtschutzmittel, Selbstbräunungsmittel, Radikalfänger, Feuchthaltemittel, Enzyme, Polymere, Melanin, mit Sauerstoff beladene asymmetrische lamellare Aggregate gemäß WO 94/00109, Kreatin, Kreatinin, Camitin, Biotin, Cardiolipin, Liponsäure, Licochalcone A, Silymarin, Silyphos, Dexpanthenol, Ursolsäure, Grüntee-Extrakte, Aminoguanidin, Ceramid-2 und Gemische davon. Dabei sind besonders bevorzugt organische Lichtschutzmittel, Radikalfänger, Kreatin, Grüntee-Extrakte, Ceramid-2 und Gemische davon.

Es ist weiterhin vorteilhaft, dem erfindungsgemäßen Kosmetikum entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester; Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Weiterhin einsetzbar als Sonnenschutzfilter sind anorganische Pigmente auf Basis von Metalloxiden, wie TiO₂, SiO₂, ZnO₂, Fe₂O₃, ZrO₂, MnO₂, Al₂O₃, die auch im Gemisch verwendet werden können.

Besonders bevorzugt als anorganische Pigmente sind agglomerierte Substrate von TiO₂ und/oder ZnO₂, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden (gemäß WO99/06012). Selbstbräunungsmittel als zusätzliche Hilfsstoffe sind u.a. Isatin, Alloxan, Ninhydrin, Glycerinaldehyd, meso-Weinsäurealdehyd, Glutaraldehyd, Erythrulose, Pirazolin-4,5-dionderivate, Dihydroxyaceton (DHA), 4,4-Dihydroxypirazolin-5-dionderivate, Glyccyhriza glabra (Süßholzwurzel), Arbutin.

Radikalfänger als zusätzliche Hilfsstoffe sind die bereits als Antioxidationsmittel genannten Substanzen sowie Ubichinon, Silymarin, Xanthin-Derivate wie Coffein, Theophyllin oder Theobromin, Ectoin, Ursolsäure, Phytinsäure, Rotweinextrakte, Ceramide, EDTA und EDTA-Derivate wie deren Alkalisalze und Gemische davon.

Die genannten Wirkstoffe und zusätzlichen Hilfsstoffe können vorteilhaft auch in verkapselter Form eingesetzt werden, z.B. in Liposomen (vorteilhaft aus Phospholipiden), in lamellaren Aggregaten oder in Cyclodextrinen oder anderen als Hilfsstoffträger bekannten Substanzen.

Das erfindungsgemäße Kosmetikum umfasst weiterhin kosmetische Hilfs- und Trägerstoffe, wie sie üblicherweise in solchen Zubereitungen verwendet werden, z.B. Wasser, Konservierungsmittel, Farbstoffe, Verdickungsmittel, Duftstoffe, Alkohole, Polyole, Ester, Ether, Elektrolyte, Gelbildner, polare und unpolare Öle, Polymere, Copolymere, Emulgatoren, Wachse, Stabilisatoren und Gemische davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie Calendulaöl, Jojobaöl, Avocadoöl, Macadamianußöl, Rizinusöl, Kakaobutter, Kokosnußöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Als Ester oder Ether sind zum Beispiel geeignet (INCI-Namen): Dipentaerythrityl Hexacaprilate/Hexacaprate/Tridecyl Ttrimellitate/Tridecyl stearate/Neopentyl Glycol Dicaprylate Dicaprate, Propylene Glycol Dioctanoate, Propylene Glycol Dicaprylate 2,30 Dicaprate, Tridecyl Stearate/Neopentyl Glycol Dicapryläte Dicaprate/Tridecyl Trimellitate, Neopentyl Glycol Dioctanoate, Isopropyl Myristate, Diisopropyl Dimer Dilinoleate, Trimethylpropane Triisostearate, Myristyl Ether, Stearyl Ether, Cetearyl Octanoate, Butyl Ether, Dicaprylyl Ether, PPG1-PEG9 Lauroyl Glycol Ether, PPG15 Stearyl Ether, PPG14 Butyl Ether, Fomblin® HC25.

Die Verwendung der erfindungsgemäßen kosmetischen Zusammensetzungen kann z.B. erfolgen in Form von Sonnencremes, Sonnengelen, Alter-sun-Produkten, Tagescremes, Nachtcremes, Masken, ,Reinigungsmilch, Körperpuder, Augenkosmetik, masken, Haarspülungen, Haarshampoos, Duschgelen, , Badeölen und in Produkten der dekorativen wie Deo-Stiften, Parfüm-Stiften, Lippenst, , Lidschattens, Kompaktproduktes wie Kompaktpukt Kompaktwachs, Rouge, Grundierung, Make-up usw.

Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Tagescreme

| **Phase A** | |
|---|---|
| Wasser | q.s. ad 100 |
| Butylene Glycol | 3,0 |
| Copolymer-845 | 0,5 |
| Xanthan Gum | 0,1 |
| Glycerine | 5,0 |

| **Phase B** | |
|---|---|
| Beheneth-10 | 3,0 |
| Cetiol CC | 4,0 |
| Shea Butter | 4,0 |
| Cyclomethicone | 2,0 |

| **Phase C** | |
|---|---|
| Dimethicone | 6,0 |

| **Phase D** | |
|---|---|
| C12-15 Alkyl Benzoate & Tribehenin & Ceramide 2 & PEG10 Rapeseed Sterol & Palmitoyl Oligopeptide (Palmitoyl-Val-Gly-Val-Ala-Pro-Gly) | 2,0 |
| Water & Hexapeptide-11 (Phe-Val-Ala-Pro-Phe-Pro) | 0,5 |
| Butylene Glycol & Water & Laureth-3 & Hydroxyethylcellulose-& Acetyl Dipeptide-1 Cetyl Ester (N-Acetyl-Tyr-Arg-Hexadodecylester) | 1,0 |
| RPF-Komplex I* | 1,0 |
| Green Tea Extract | 0,5 |
| Orange Extract | 0,1 |
| Kaolin (D₅₀ 240 µm) | 1,0 |
| TiO₂ (D₅₀ 20 µm) | 0,1 |
| Parfüm | 0,3 |
| Konservierungsmittel | 0,2 |
| Hyaluronic Acid | 0,2 |

| | |
|---|---|
| * bestehend aus Wasser, 1 % Gelbildner, 7,5 % Phospholipide, 2 - Quebracho-Extrakt und 1 % Seidenraupen-Extrakt (gemäß WO 99/66881), bezogen auf das Gesamtgewicht des Antioxidationsmittels. | |

Die Phasen A und B werden separat auf 75 °C erwärmt. Dann wird die Phase B in Phase A unter Rühren eingebracht. Nach dem Abkühlen auf etwa 50 °C erfolgt die Zugabe der Phase C ebenfalls unter Rühren. Schließlich wird die Phase D unter Rühten bei etwa 35 °C zugegeben und die Mischung homogenisiert.

### Beispiel 2 Nachtcreme

| **Phase A** | |
|---|---|
| Wasser | q.s. ad 100 |
| Carhomer | 0,25 |
| Xanthan Gum | 0,05 |
| Glycerine | 5,0 |

| **Phase B** | |
|---|---|
| Behenyl Alcohol | 1, 0 |
| Polyglyceryl-3 Diisostearate | 0,5 |
| Dicaprylyl Carbonate | 1,0 |
| Tocopherol | 5,0 |
| Silicone | 3,0 |

| **Phase C** | |
|---|---|
| Jojoba Oil | 5,0 |

| **Phase D** | |
|---|---|
| C12-15 Alkyl Benzoate & Tribehenin & Ceramide 2 & PEG10 Rapeseed Sterol & Palmitoyl Oligopeptide (Palmitoyl-Val-Gly-Val-Ala-Pro-Gly) | 2,0 |
| Water & Hexapeptide-11 (Phe-Val-Ala-Pro-Phe-Pro) | 0,5 |
| Butylene Glycol & Water & Laureth-3 & HydroxyEthylcellulose & Acetyl Dipeptide-1 Cetyl Ester (N-Acetyl-Tyr-Arg-Hexadodecylester) | 1,0 |
| RPF-Komplex I* | 0,3 |
| Retinol | 0,5 |
| Kaolin (D₅₀ 200 µm) | 1,0 |
| TiO₂ (D₅₀ 20 µm) | 0,1 |
| Parfüm | 0,1 |
| Konservierungsmittel | 0,1 |
| Hyaluronic Acid | 0,15 |

| | |
|---|---|
| * wie Beispiel 1 | |

### Beispiel 3 Tagescreme II

Die Phasen A bis C entsprechend denen von Beispiel 1.

### Phase D

| | |
|---|---|
| C12-15 Alkyl Benzoate & Tribehenin & Ceramide 2 & PEG10 Rapeseed Sterol & Palmitoyl Oligopeptide (Palmitoyl-Val-Gly-Val-Ala-Pro-Gly) | 0,3 |
| Water & Hexapeptide-11 (Phe-Val-Ala-Pro-Phe-Pro) | 3,0 |
| Butylene Glycol & Water & Laureth-3 & Hydroxyethylcellulose- & Acetyl Dipeptide-1 Cetyl Ester (N-Acetyl-Tyr-Arg-Hexadodecylester) | 0,2 |
| RPF-Komplex I* | 0,4 |
| Green Tea Extract | 0,2 |
| Orange Extract | 0,2 |
| Kaolin (D₅₀ 240 µm) | 0,5 |
| TiO₂ (D₅₀ 20 µm) | 0,1 |
| Parfüm | 0,3 |
| Konservierungsmittel | 0,2 |
| Hyaluronic Acid | 1,5 |

| | |
|---|---|
| * siehe Beispiel 1 | |

### Beispiel 4 Tagescreme III

Die Phasen A bis C entsprechend denen von Beispiel 1.

### Phase D

| | |
|---|---|
| C12-15 Alkyl Benzoate & Tribehenin & Ceramide 2 & PEG10 Rapeseed Sterol & Palmitoyl Oligopeptide (Palmitoyl-Val-Gly-Val-Ala-Pro-Gly) | 4,5 |
| Water & Hexapeptide-11 (Phe-Val-Ala-Pro-Phe-Pro) | 6,0 |
| Butylene Glicol & Water & Laureth-3 & HydroxyEthylcellulose- & Acetyl Dipeptide-1 Cetyl Ester (N-Acetyl-Tyr-Arg-Hexadodecylester) | 0,8 |
| RPF-Komplex I* | 1,2 |
| Green Tea Extract | 0,6 |
| Orange Extract | 0,2 |
| Kaolin (D₅₀ 240 µm) | 1,8 |
| TiO₂ (D₅₀ 20 µm) | 0,2 |
| Parfüm | 0,3 |
| Konservierungsmittel | 0,2 |
| Hyaluronic Acid | 8,0 |

| | |
|---|---|
| * siehe Beispiel 1 | |

### Beispiel 5 Anwender-Test

Es wurde ein Anwendertest mit 40 Teilnehmerinnen im Alter von 41 bis 60 Jahren durchgeführt. 20 Teilnehmerinnen (Gruppe A) applizierten eine Creme A gemäß Beispiel 1 im Gesicht, insbesondere rund um die Augenpartie. Die andere Gruppe (B) applizierten eine Creme B. Die Creme B enthielt die gleichen Bestandteile wie in Beispiel 1 genannt, jedoch ohne das Hexapeptid Phe-Val-Ala-Pro-Phe-Pro, das Dipeptid Tyr-Arg und die Pigmente Kaolin und TiO₂.

Die Cremes wurden zweimal täglich morgens und abends mit einer durchschnittlichen Menge von 2-4 mg/cm² aufgetragen. Es erfolgte eine computerisierte Auswertung über digitalisierte Aufnahmen von den Augenwinkeln der Probandinnen. Die Faltenreduzierung der feinen Augenwinkelfalten wurde in Prozent festgestellt.

Die Ergebnisse sind in Tabelle 1 dargestellt.

**Tabelle 1**

| | Faltenreduzierung [%] | |
|---|---|---|
| | Gruppe A | Gruppe B |
| Behandlungs- Beginn | 0 | 0 |
| nach 3 Tagen | 22-27 | 3-4 |
| 7 Tagen | 34-39 | 8-10 |
| 10 Tagen | 44-49 | 10-11 |
| 20 Tagen | 45-51 | 17-21 |
| 30 Tagen | 44-51 | 23-28 |
| 60 Tagen | 45-53 | 20-31 |

Tabelle 1 zeigt deutlich die Überlegenheit der Creme A mit dem erfindungsgemäßen Komplex gegenüber der Einzelanwendung eines Hexapeptids (Val-Gly-Val-Ala-Pro-Gly) in der Creme B. Während die Behandlung mit einer Creme B zu einem erwarteten Anstieg der Faltenreduzierung im Zeitraum bis zu 2 Monaten von etwa 30 % führt, zeigt die erfindungsgemäße Creme A gemäß Beispiel 1 bereits in den ersten Tagen einen überraschenden Anstieg, der nach 10 Tagen bis auf nahe 50 % führt und danach kaum weiter steigt. Dies ist ein signifikantes und überraschendes Ergebnis.

## Patentansprüche

1. Antifalten-Kosmetikum auf Peptidbasis, **dadurch gekennzeichnet, dass** es ein Peptidgemisch aus zwei unterschiedlichen Hexapeptiden mit den Aminosäuresequenzen Val-Gly-Val-Ala-Pro-Gly und Phe-Val-Ala-Pro-Phe-Pro, ein Dipeptid mit der Sequenz Tyr-Arg, und einen Gehalt an Hyaluronsäure, Antioxidationsmittel und Pigment oder Pigmentgemisch mit einer Teilchengröße im Bereich von 0,5-300 µm umfasst sowie kosmetische Trägerstoffe, Hilfsstoffe und Gemische davon.

2. Antifalten-Kosmetikum nach Anspruch 1, **dadurch gekennzeichnet, dass** das Hexapeptid Val-Gly-Val-Ala-Pro-Gly ein Palmitoyl-VGVAPG-Peptid ist.

3. Antifalten-Kosmetikum nach Anspruch 1 oder 2, **dadurch gekennzeichnet, dass** das Dipeptid ein N-Acetyl-Tyr-Arg-Hexadodecylester ist.

4. Antifalten-Kosmetikum nach einem der Ansprüche 1 bis 3, **dadurch gekennzeichnet, dass** das Pigment aus der Gruppe ausgewählt ist, bestehend aus Titanoxid, Zinkoxid, Aluminiumoxid, Eisenoxide, Manganoxid, Glimmer, Kaolin, manganhaltige Tone, Calciumcarbonat, Talkum, Glimmer-Titanoxid, Glimmer-Titanoxid-Eisenoxid, beschichtete Pigmente und Gemischen davon, vorzugsweise aus Titanoxid, Zinkoxid, Eisenoxide, Kaolin, Talkum und Gemischen davon.

5. Antifalten-Kosmetikum nach einem der Ansprüche 1 bis 4, **dadurch gekennzeichnet, dass** das Pigment oder Pigmentgemisch unterschiedliche Teilchengrößen, die sich um mindestens 50 µm bis 250 µm unterscheiden, aufweist.

6. Antifalten-Kosmetikum nach einem der Ansprüche 1 bis 5, **dadurch gekennzeichnet, dass** das Antioxidationsmittel aus der Gruppe ausgewählt ist, bestehend aus Ascorbinsäure, Ascorbylacetat, Ascorbylphosphat, Ascorbylpalmitat, Magnesiumascorbyl-phosphat, Retinol, Retinolpalmitat, Folsäure, N5-Formyltetrahydrofolsäure, N10-Formyltetrahydrofolsäure, Folsäurekonjugate mit 2-8 Glutamylresten, Tocopherol, Tocopherylacetat, Tocopheryllactat, Tocopherylpalmitat, Tocopherylsuccinat, Tocopherylnicotinat, Tocopheryllinoleat, Flavone, Flavonoide, Histidin, Glycin, Tyrosin, Tryptophan, cis-Urocaninsäure, trans-Urocaninsäure, D,L-Carnosin, D-Carnosin, L-Carnosin, α-Carotin, ß-Carotin, Lycopin, Harnsäure, Citronensäure, Milchsäure, Apfelsäure, Palmitinsäure, Phytinsäure, Lactoferrin, Ferulasäure, Glutathion, Cystein, Cystin, Pflanzenextrakten, Gemischen von Pflanzenextrakten und Gemischen von zwei oder mehreren dieser Antioxidationsmittel.

7. Antifalten-Kosmetikum nach einem der Ansprüche 1 bis 6, **dadurch gekennzeichnet, dass** das Antioxidationsmittel ein Gemisch von Pflanzenextrakten und Vitaminen ist.

8. Antifalten-Kosmetikum nach Anspruch 7, **dadurch gekennzeichnet, dass** das Antioxidationsmittel ein Gemisch ist mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie mit einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

9. Antifalten-Kosmetikum nach einem der Ansprüche 1 bis 8, **dadurch gekennzeichnet, dass** der Anteil des Peptidgemisches im Bereich von 0,5 bis 18 Gew-% liegt, vorzugsweise 0,9-5 Gew-%, bezogen auf das Gesamtgewicht des Kosmetikums.

10. Antifalten-Kosmetikum nach einem der Ansprüche 1 bis 9, **dadurch gekennzeichnet, dass** der Anteil des Peptids Val-Gly-Val-Ala-Pro-Gly im Bereich von 0,01 bis 5 Gew-% liegt, vorzugsweise 0,1-3 Gew-%, bezogen auf das Gesamtgewicht des Kosmetikums.

11. Antifalten-Kosmetikum nach einem der Ansprüche 1 bis 10, **dadurch gekennzeichnet, dass** der Anteil des Peptids Phe-Val-Ala-Pro-Phe-Pro im Bereich von 0,01 bis 8 Gew-% liegt, vorzugsweise 0,1-3 Gew-%, bezogen auf das Gesamtgewicht des Kosmetikums.

12. Antifalten-Kosmetikum nach einem der Ansprüche 1 bis 11, **dadurch gekennzeichnet, dass** der Anteil des Peptids Tyr-Arg im Bereich von 0,01 bis 5 Gew-% liegt, vorzugsweise 0,1-3 Gew-%, bezogen auf das Gesamtgewicht des Kosmetikums.

13. Antifalten-Kosmetikum nach einem der Ansprüche 1 bis 12, **dadurch gekennzeichnet, dass** der Anteil des Antioxidationsmittels im Bereich von 0,01 bis 5 Gew-% liegt, vorzugsweise 0,3-2 Gew-%, bezogen auf das Gesamtgewicht des Kosmetikums.

14. Antifalten-Kosmetikum nach einem der Ansprüche 1 bis 13, **dadurch gekennzeichnet, dass** der Anteil der Hyaluronsäure im Bereich von 0,01 bis 10 Gew-% liegt, vorzugsweise 0,5-3 Gew-%, bezogen auf das Gesamtgewicht des Kosmetikums.

15. Antifalten-Kosmetikum nach einem der Ansprüche 1 bis 14, **dadurch gekennzeichnet, dass** der Anteil des Pigments im Bereich von 0,05 bis 2 Gew-% liegt, vorzugsweise 0,5-1,2 Gew-%, bezogen auf das Gesamtgewicht des Kosmetikums.

16. Antifalten-Kosmetikum nach einem der Ansprüche 1 bis 15, **dadurch gekennzeichnet, dass** die Hilfsstoffe ausgewählt sind aus der Gruppe, bestehend aus anorganischen Lichtschutzmitteln, organischen Lichtschutzmitteln, Selbstbräunungsmitteln, Radikalfängern, Feuchthaltemitteln, Enzymen, Polymeren, Melanin, mit Sauerstoff beladenen asymmetrischen lamellaren Aggregaten gemäß WO 94/00109, Kreatin, Kreatinin, Camitin, Biotin, Cardiolipin, Liponsäure, Licochalcone A, Silymarin, Silyphos, Dexpanthenol, Ursolsäure, Grüntee-Extrakten, Aminoguanidin, Ceramid-2 und Gemischen davon.

## Claims

1. An anti-wrinkle cosmetic on a peptide base, **characterized in that** it comprises a peptide mixture including two different hexapeptides whose amino acid sequences are Val-Gly-Val-Ala-Pro-Gly and Phe-Val-Ala-Pro-Phe-Pro, a dipeptide whose sequence is Tyr-Arg, and a content of hyaluronic acid, antioxidant and pigment or pigment mixture having a particle size in the range of 0.5-300 µm, as well as cosmetic carriers, auxiliaries and mixtures thereof.

2. The anti-wrinkle cosmetic according to claim 1, **characterized in that** the Val-Gly-Val-Ala-Pro-Gly hexapeptide is a palmitoyl-VGVAPG peptide.

3. The anti-wrinkle cosmetic according to claim 1 or 2, **characterized in that** the dipeptide is a N-acetyl-Tyr-Arg hexadodecylester.

4. The anti-wrinkle cosmetic according to any one of claims 1 to 3, **characterized in that** the pigment is selected from the group consisting of titanium oxide, zinc oxide, aluminium oxide, iron oxides, manganese oxide, mica, kaolin, clays containing manganese, calcium carbonate, talc, mica-titanium oxide, mica-titanium oxide-iron oxide, coated pigments and mixtures thereof, preferably of titanium oxide, zinc oxide, iron oxides, kaolin, talc and mixtures thereof.

5. The anti-wrinkle cosmetic according to any one of claims 1 to 4, **characterized in that** the pigment or pigment mixture comprises different particle sizes, which differ by at least 50 µm to 250 µm.

6. The anti-wrinkle cosmetic according to any one of claims 1, to 5, **characterized in that** the antioxidant is selected from the group consisting of ascorbic acid, ascorbyl acetate, ascorbyl phosphate, ascorbyl palmitate, magnesium ascorbyl phosphate, retinol, retinol palmitate, folic acid, N5-formyltetrahydrofolic acid, N10-formyltetra-hydrofolic acid, folic acid conjugates having 2-8 glutamyl residues, tocopherol, tocopheryl acetate, tocopheryl lactate, tocopheryl palmitate, tocopheryl succinate, tocopheryl nicotinate, tocopheryl linoleate, flavones, flavonoids, histidine, glycine, tyrosine, tryptophan, cis-urocanic acid, trans-urocanic acid, D,L-carnosine, D-carnosine, L-carnosine, α-carotene, β-carotene, lycopene, uric acid, citric acid, lactic acid, malic acid, palmitic acid, phytic acid, lactoferrin, ferulic acid, glutathione, cysteine, cystine, plant extracts, mixtures of plant extracts and mixtures of two or more of these antioxidants.

7. The anti-wrinkle cosmetic according to any one of claims 1 to 6, **characterized in that** the antioxidant is a mixture of plant extracts and vitamins.

8. The anti-wrinkle cosmetic according to claim 7, **characterized in that** the antioxidant is a mixture containing an amount of a product obtained by extracting the bark of Quebracho blanco and subsequent enzymatic hydrolysis, which contains at least 90 % by weight of pro-anthocyanidine oligomers and at most 10 % by weight of gallic acid, in micro-capsules, and including a silkworm extract obtained by extraction, which contains the peptide cecropine, amino acids and a vitamin mixture, and a non-ionic, cationic or anionic hydrogel or mixture of hydrogels, and one or more phospholipid(s), and water.

9. The anti-wrinkle cosmetic according to any one of claims 1 to 8, **characterized in that** the content of the peptide mixture contained therein is in the range from 0.5 to 18 % by weight, preferably 0.9-5 % by weight, relative to the total weight of the cosmetic.

10. The anti-wrinkle cosmetic according to any one of claims 1 to 9, **characterized in that** the content of the Val-Gly-Val-Ala-Pro-Gly peptide contained therein is in the range from 0.01 to 5 % by weight, preferably 0.1-3 % by weight, relative to the total weight of the cosmetic.

11. The anti-wrinkle cosmetic according to any one of claims 1 to 10, **characterized in that** the content of the Phe-Val-Ala-Pro-Phe-Pro peptide contained therein is in the range from 0.01 to 8 % by weight, preferably 0.1-3 % by weight, relative to the total weight of the cosmetic.

12. The anti-wrinkle cosmetic according to any one of claims 1 to 11, **characterized in that** the content of the Tyr-Arg peptide contained therein is in the range from 0.01 to 5 % by weight., preferably 0.1-3 % by weight, relative to the total weight of the cosmetic.

13. The anti-wrinkle cosmetic according to any one of claims 1 to 12, **characterized in that** the content of the antioxidant contained therein is in the range from 0.01 to 5 % by weight, preferably 0.3-2 % by weight, relative to the total weight of the cosmetic.

14. The anti-wrinkle cosmetic according to any one of claims 1 to 13, **characterized in that** the content of hyaluronic acid contained therein is in the range from 0.01 to 10 % by weight, preferably 0.5-3 % by weight, relative to the total weight of the cosmetic.

15. The anti-wrinkle cosmetic according to any one of claims 1 to 14, **characterized in that** the content of pigment contained therein is in the range from 0.05 to 2 % by weight, preferably 0.5-1.2 % by weight, relative to the total weight of the cosmetic.

16. The anti-wrinkle cosmetic according to any one of claims 1 to 15, **characterized in that** the auxiliaries are selected from the group consisting of inorganic sunscreens, organic sunscreens, self-tanning agents, radical scavengers, moisturizers, enzymes, polymers, melanin, asymmetric lamellar aggregates loaded with oxygen according to WO 94/00109, creatine, creatinine, carnitine, biotin, cardiolipin, lipoic acid, licochalcone A, silymarin, silyphos, dexpanthenol, ursolic acid, green tea extracts, aminoguanidine, ceramide-2 and mixtures thereof.

## Revendications

1. Cosmétique anti-rides à base de peptides, **caractérisé en ce qu'**il comprend un mélange de peptides composé de deux hexapeptides différents avec les séquences d'acides aminés val-gly-val-ala-pro-gly et phe-val-ala-pro-phe-pro, un dipeptide avec la séquence tyr-arg, et une teneur en acide hyaluronique, en anti-oxydant et en pigment ou en mélange de pigments avec une taille de particules dans la plage de 0,5-300 µm, ainsi que des substances porteuses et auxiliaires, et des mélanges de ceux-ci.

2. Cosmétique anti-rides selon la revendication 1, **caractérisé en ce que** l'hexapeptide val-gly-val-ala-pro-gly est un peptide palmitate de VGVAPG.

3. Cosmétique anti-rides selon la revendication 1 ou 2, **caractérisé en ce que** le dipeptide est un hexadodecylester n-acetyl-tyr-arg.

4. Cosmétique anti-rides selon l'une des revendications 1 à 3, **caractérisé en ce que** le pigment est sélectionné dans un groupe se composant d'oxyde de titane, d'oxyde de zinc, d'oxyde d'aluminium, d'oxyde de fer, d'oxyde de manganèse, de mica, de kaolin, d'argiles contenant du manganèse, de carbonate de calcium, de talc, de mica avec oxyde de titane, de mica avec oxyde de fer et oxyde de titane, de pigments recouverts et de leurs mélanges, se composant de préférence d'oxyde de titane, d'oxyde de zinc, d'oxyde de fer, de kaolin, de talc et de leurs mélanges.

5. Cosmétique anti-rides selon l'une des revendications 1 à 4, **caractérisé en ce que** le pigment ou le mélange de pigments présente des tailles de particules différentes, avec un écart d'au minimum 50 pm à 250 pm.

6. Cosmétique anti-rides selon l'une des revendications 1 à 5, **caractérisé en ce que** l'anti-oxydant est sélectionné dans un groupe contenant l'acide ascorbique, l'acétate d'ascorbyle, le phosphate d'ascorbyle, le palmitate d'ascorbyle, le phosphate d'ascorbyle de magnésium, le rétinol, le palmitate de rétinol, l'acide folique, l'acide N5-formyltétrahydrofolique, l'acide N10-formyltétrahydrofolique, les conjugaisons d'acide folique avec 2-8 restes de glutamyle, le tocophérol, l'acétate de tocophéryle, le lactate de tocophéryle, le palmitate de tocophéryle, le succinate de tocophéryle, le nicotinate de tocophéryle, le linoléate de tocophéryle, les flavones, les flavonoïdes, l'histidine, la glycine, la tyrosine, le tryptophane, l'acide cis-urocanique, l'acide trans-urocanique, la D, L-carnosine, la D-carnosine, la L-carnosine, l'alpha-carotène, le bêta-carotène, le lycopène, l'acide urique, l'acide citrique, l'acide lactique, l'acide malique, l'acide palmitique, l'acide physique, la lactoferrine, l'acide férulique, le gluthation, la cystéine, la cystine, des extraits de plantes, des mélanges d'extraits de plantes et ùes mélanges de deux ou plusieurs de ces antioxydants.

7. Procédé selon l'une des revendications précédentes 1 à 6, **caractérisé en ce que** l'antioxydant est un mélange d'extraits de plantes et de vitamines.

8. Cosmétique anti-rides selon la revendications 7, **caractérisé en ce que** l'antioxydant est un mélange avec une teneur en un produit obtenu par extraction de l'écorce de quebracho blanc et de l'hydrolyse enzymatique subséquente, lequel contient au minimum 90 % poids d'oligomères proanthocyanidiques et au maximum 10 % poids d'acide gallique, en microcapsules, ainsi qu'un extrait de ver à soie obtenu par extraction, lequel contient le peptide cécropine, des acides aminés et un mélange de vitamines, et un hydrogel ou mélange d'hydrogels non ionique, cationique ou anionique, et un ou plusieurs phospholipides, et de l'eau.

9. Cosmétique anti-rides selon l'une des revendications 1 à 8, **caractérisé en ce que** la part du mélange de peptides se trouve dans une plage de 0,5 à 18 % poids, de préférence 0,9-5 % poids, relativement au poids total du cosmétique.

10. Cosmétique anti-rides selon l'une des revendications 1 à 9, **caractérisé en ce que** la part du peptide val-gly-val-ala-pro-gly se trouve dans une plage de 0,01 à 5 % poids, de préférence 0,1-3 % poids, relativement au poids total du cosmétique.

11. Cosmétique anti-rides selon l'une des revendications 1 à 10, **caractérisé en ce que** la part du peptide phe-val-ala-pro-phe-pro se trouve dans une plage de 0,01 à 8 % poids, de préférence 0,1-3 % poids, relativement au poids total du cosmétique.

12. Cosmétique anti-rides selon l'une des revendications 1 à 11, **caractérisé en ce que** la part du peptide tyr-arg se trouve dans une plage de 0,01 à 5 % poids, de préférence 0,1-3 % poids, relativement au poids total du cosmétique.

13. Cosmétique anti-rides selon l'une des revendications 1 à 12, **caractérisé en ce que** la part de l'antioxydant se trouve dans une plage de 0,01 à 5 % poids, de préférence 0,3-2 % poids, relativement au poids total du cosmétique.

14. Cosmétique anti-rides selon l'une des revendications 1 à 13, **caractérisé en ce que** la part de l'acide hyaluronique se trouve dans une plage de 0,01 à 10 % poids, de préférence 0,5-3 % poids, relativement au poids total du cosmétique.

15. Cosmétique anti-rides selon l'une des revendications 1 à 14, **caractérisé en ce que** la part du pigment se trouve dans une plage de 0,05 à 2 % poids, de préférence 0,5-1,2 % poids, relativement au poids total du cosmétique.

16. Cosmétique anti-rides selon l'une des revendications 1 à 15, **caractérisé en ce que** les substances auxiliaires sont sélectionnées dans un groupe se composant de moyens photoprotecteurs anorganiques, d'agents photoprotecteurs organiques, d'agents autobronzants, de fixateurs de radicaux, d'agents humectants, d'enzymes, de polymères, de mélanine, d'agrégats lamellaires asymétriques chargés en oxygène conformément à WO 94/00109, de créatine, de créatinine, de carnitine, de biotine, de cardiolipine, d'acide lipoïque, de licochalcone A, de silymarine, de silyphos, de dexpanthénol, d'acide ursolique, d'extraits de thé vert, d'aminoguanidine, de céramide 2 et de mélanges de ceux-ci.
